# EUROPÄISCHE PATENTSCHRIFT

(11) **EP 3 348 243 B1**
(45) Veröffentlichungstag und Bekanntmachung des Hinweises auf die Patenterteilung: **05.05.2021**
(21) Anmeldenummer: 18151063.7
(22) Anmeldetag: 10.01.2018
(51) Int. Cl.: A61F 9/02

(54) **BRILLE FÜR DIE AUFNAHME VON MINDESTENS EINEM STRAHLENSCHUTZMATERIAL**
SPECTACLES FOR HOLDING AT LEAST ONE RADIATION PROTECTION MATERIAL
LUNETTES POUR LA RÉCEPTION D'AU MOINS UN MATÉRIAU DE PROTECTION CONTRE LES RAYONNEMENTS

(30) Priorität: 12.01.2017 DE 102017200437
(43) Veröffentlichungstag der Anmeldung: 18.07.2018
(73) Patentinhaber: MAVIG GmbH, 81829 München (DE)
(72) Erfinder: Ballsieper, Barbara, 81829 München (DE); Schmid, Martin, 81829 München (DE)
(74) Vertreter: Vossius & Partner Patentanwälte Rechtsanwälte mbB

(56) Entgegenhaltungen:
- EP-A2- 1 623 690
- DE-A1-102006 028 958
- TW-U- M 528 439
- US-A- 2 204 631
- US-A- 4 021 862
- US-A- 5 422 684
- US-A1- 2008 273 165

## Beschreibung

Die Erfindung betrifft eine Brille für die Aufnahme von mindestens einem Strahlenschutzmaterial, das die Augen eines Patienten vor augenschädlicher Strahlung, insbesondere β-Strahlung und/oder Röntgenstrahlung und/oder Gammastrahlen, schützt. Insbesondere betrifft die vorliegende Erfindung die Verwendung der erfindungsgemäßen Brille als Strahlenschutzbrille für den Schutz von Augen eines Patienten vor augenschädlicher Strahlung, insbesondere β-Strahlung und/oder Röntgenstrahlung und/oder Gammastrahlung.

Aus dem Stand der Technik sind verschiedene Strahlenschutzbrillen bekannt.

Die DE 31 16 760 A1 betrifft eine Strahlenschutzbrille mit einem flexiblen Tragkörper, dessen Rand gut am Kopf des Brillenträgers anliegt und der eine Laserstrahlen-Abschirmung aufweist, die von der Seite her einfallende Strahlung daran hindert, in die Augen des Brillenträgers einzudringen. Ferner umfasst die Strahlenschutzbrille zumindest eine Metallschicht, die sich bis in den Bereich des zur Anlage am Kopf des Brillenträgers bestimmten Randes des Tragkörpers erstreckt. Die Metallschicht kann dabei eine Metallfolie aus Aluminium sein, die in den Tragkörper eingebettet ist.

Die US 4,024,405 A betrifft eine Schutzbrille gegen Röntgenstrahlung, wobei die Schutzbrille an die Kopfform um die Augen angepasst ist und somit auch vor seitlich einfallenden Röntgenstrahlen schützt. Hierzu wird ein Bleifilm in ein Kunststoffmaterial eingearbeitet.

Die DE 94 14 879 U1 und DE 93 14 811 U1 betreffen eine Strahlenschutzbrille, insbesondere zum Schutz gegen Laserstrahlung, die aus mindestens einem Schutzfilter und einer Brillenfassung besteht. Der Inneneinsatz besteht dabei aus einem hautfreundlichen Elastomer, welches in der Lage ist auf sie treffende Laserstrahlung zu absorbieren.

Die US 5,016,292 A betrifft eine Sicherheitsbrille, die z.B. vor UV-, Gamma- und Röntgenstrahlung schützt. Die Brille ist ausgelegt, die Augen vor Strahlung aus allen möglichen Richtungen zu schützen. Die Linse 8 der Sicherheitsbrille besteht dabei aus einem mit Blei versetzten Polymer.

Die US 4,021,862 A betrifft eine Strahlenschutzbrille gegen ionisierende Strahlung aus verschiedenen Richtungen, wobei die Sichtfenster und die restliche Abschirmung der Brille aus Materialien bestehen, die mit Blei versetzt sind.

Die DD 227 267 A1 betrifft eine Schutzbrille mit längenverstellbarer Brücke und besonderer Abdichtung im Nasenbereich, insbesondere zum Schutz gegen Strahlung. Die Brillenfassungshälften tragen Nasenstegaufbauten, die in einem Viertelbogen auslaufen und in einem flächigen Anschlag enden.

Zusammenfassend sind mehrere Strahlenschutzbrillen aus dem Stand der Technik bekannt. Allerdings sind alle bekannten Strahlenschutzbrillen zum Schutz einer ganz bestimmten Strahlung ausgelegt. Die Strahlenschutzbrillen für den Laserbereich eignen sich durch die viel höhere Wellenlänge von Laserstrahlung im Vergleich zur Röntgenstrahlung nicht für den Einsatz zum Schutz vor Röntgenstrahlung.

Mit anderen Worten, keine der vorgenannten Strahlenschutzbrillen ist ausgelegt, um für verschiedene Einsatzzwecke verwendet zu werden.

Weiterhin sind auf dem Markt CT-Strahlenschutzvorrichtungen für die Augen erhältlich, die beispielsweise von den Firmen SOMATEX Medical Technologies ("CT-Eye-ProteX"), F & L Medical Products ("AttenuRad CT Eye Shield") und Kemmetech Ltd. ("GreyShield Eye Protection Shield") vertrieben werden. Diese Strahlenschutzvorrichtungen bedecken die Augen komplett, sodass der Patient keine Möglichkeit hat, während der Untersuchung etwas zu lesen. Ferner liegen diese Strahlenschutzvorrichtungen lediglich auf den Augen auf und ein stabiler Halt kann somit nicht garantiert werden, wenn der Patient sich während der Untersuchung bewegt.

Ferner ist keine der von den vorgenannten Firmen erhältlichen Strahlenschutzvorrichtungen ausgelegt, um für verschiedene Einsatzzwecke verwendet zu werden.

Es ist eine Aufgabe der vorliegenden Erfindung, eine Strahlenschutzbrille bereitzustellen, die die Nachteile des Stands der Technik vermeidet bzw. verringert. Insbesondere ist es eine Aufgabe der vorliegenden Erfindung, eine Strahlenschutzbrille bereitzustellen, die für verschiedene Strahlenarten verwendet werden kann und für die Anwendung in der Computertomographie und der Radiotherapie geeignet ist.

Diese Aufgaben werden durch die Merkmale des Anspruchs 1 gelöst.

Die abhängigen Patentansprüche beziehen sich auf weitere Aspekte der Erfindung.

Gemäß einem Aspekt der vorliegenden Offenbarung wird eine Brille, insbesondere eine Strahlenschutzbrille, für die Aufnahme von mindestens einem Strahlenschutzmaterial, das die Augen eines Patienten vor augenschädlicher Strahlung, insbesondere β-Strahlung und/oder Röntgenstrahlung und/oder Gammastrahlen, schützt, bereitgestellt. Die Brille aufweisend: zwei Brillenfassungen, die jeweils eine durchgängig umlaufende Seitenwandstruktur aufweisen, die einen Bereich um das jeweilige Auge des Patienten vollständig umschließen, ein längenverstellbares Verbindungselement zwischen den zwei Brillenfassungen im Bereich der Nase des Patienten, um den Abstand der zwei Brillenfassungen zueinander zu verändern, und mindestens ein längenverstellbares Halteelement, um die zwei Brillenfassungen am Kopf des Patienten zu fixieren, wobei die Seitenwandstrukturen konfiguriert sind, mindestens ein Strahlenschutzmaterial im Wesentlichen vollumfänglich aufzunehmen.

Die vorliegende Erfindung geht von dem Grundgedanken aus, eine Brille bereitzustellen, die beliebig mit Strahlenschutzmaterial beladen werden kann. Dies bedeutet konkret, die Grundform der Strahlenschutzbrille ist für den Einsatz als Betaschutzbrille ebenso geeignet, wie für den Einsatz als Röntgenschutzbrille oder als Gammastrahlenschutzbrille. Dies erlaubt es, die Strahlenschutzbrille, falls gewünscht, auch für verschiedene Strahlenarten zu verwenden.

Das Strahlenschutzmaterial wird im Wesentlichen vollumfänglich von der Seitenwandstruktur aufgenommen. Mit anderen Worten, die Seitenwandstruktur ist konfiguriert, um mindestens ein Strahlenschutzmaterial möglichst vollumfänglich aufzunehmen. Somit wird seitlich einfallende Strahlung effektiv vom Auge abgeschirmt. Dadurch kann augenschädliche Strahlung aus einem möglichst großen Bereich um die Augen, vor allem aus den seitlichen Richtungen, hinreichend abgeschirmt werden.

Die Seitenwandstruktur weist jeweils eine innere und eine äußere Seitenwand auf, die vorzugsweise über eine Basiswand an einem Ende der Seitenwandstruktur verbunden sind.

Die innere und die äußere Seitenwand haben einen vorbestimmten Abstand in Querrichtung zur Blickrichtung des Patienten, um einen Zwischenraum zu bilden, wobei der Zwischenraum konfiguriert ist, das mindestens eine Strahlenschutzmaterial aufzunehmen, das vorzugsweise derart ausgebildet ist, den Zwischenraum in Längsrichtung vollständig auszufüllen.

Der vorbestimmte Abstand, d.h. der Zwischenraum, kann beispielsweise 0,5 mm, vorzugsweise 1 mm und besonders bevorzugt 2 mm betragen.

Ferner kann der Zwischenraum in Umfangsrichtung der Brillenfassungen variieren.

Der Zwischenraum kann von der Brillenoberseite (distales Ende) zur Brillenunterseite (proximales Ende) konisch ausgestaltet sein. Dies ist sowohl für die Spritzgussfertigung wie auch die Stabilität von Vorteil.

Wie vorstehend beschrieben ist es bevorzugt, dass das Strahlenschutzmaterial den Zwischenraum in Längsrichtung vollständig ausfüllt. Die Längsrichtung steht dabei senkrecht auf dem Abstandsvektor zwischen der inneren und der äußeren Seitenwand. Die vollständige Ausfüllung des Zwischenraums in Längsrichtung erlaubt dabei einen möglichst effektiven Schutz vor seitlich einfallender Strahlung.

Das Strahlenschutzmaterial kann den Zwischenraum in Querrichtung zur Längsrichtung teilweise oder vollständig ausfüllen. Der Abstand zwischen der inneren und der äußeren Seitenwand wird vorzugsweise nicht vollständig mit Strahlenschutzmaterial ausgefüllt.

Nicht erfindungsgemäß wird das mindestens eine Strahlenschutzmaterial an der oben genannten Seitenwandstruktur aufgebracht werden, ohne dass ein Zwischenraum mittels einer inneren und einer äußeren Seitenwand gebildet wird. Das Strahlenschutzmaterial kann beispielsweise auf die Seitenwandstruktur geklebt werden.

Der spezifische Einsatzzweck der Strahlenschutzbrille wird durch die Beladung des Zwischenraums (Spalt zwischen innerer und äußerer Seitenwand) mit dem Strahlenschutzmaterial festgelegt.

Das mindestens eine Strahlenschutzmaterial kann eine erste Materialschicht aus einem Material mit niedriger Kernladungszahl Z, insbesondere Al, und/oder eine zweite Materialschicht aus einem Material mit hoher Kernladungszahl Z, insbesondere Blei oder Bleiersatzmaterialien, wie Bismuth, Wolfram, Tantal oder Verbindungen dieser Metalle, aufweisen. Die erste Materialschicht und die zweite Materialschicht sind vorzugsweise in Form einer Verbundschicht, in Form einer dünnen Folie oder als Pulverform in einem Matrixmaterial dispergiert oder als Flüssigkeit zum Gießen oder Spritzen vorgesehen.

Insbesondere die Ausgestaltung mit einem Zwischenraum erlaubt die Einbringung der Materialschicht als Flüssigkeit (auch als Füllung oder Füllmaterial bezeichnet) im Rahmen eines Gieß- oder Spritzverfahrens. Der Zwischenraum wird in diesem Fall vollständig mit dem Füllmaterial befüllt, im Wesentlichen ohne Lufteinschlüsse.

Die Verwendung des oben genannten Gieß- oder Spritzverfahrens zeichnet sich unter anderem durch sein einfaches Prinzip aus. Da die äußere Seitenwand mit der inneren Seitenwand eine Kavität (Zwischenraum) bildet, bietet sich die Möglichkeit der Einbringung beispielsweise eines flüssigen Polymers, das mit Strahlenschutzadditiven beladen ist, in die Kavität an. Dieses Verfahren ist produktionstechnisch sehr einfach und kann prinzipiell analog zu typischen Verfahren in der Elektronikindustrie zum Vergießen von elektronischen Schaltungen mit Epoxidharzen zur Befüllung der Kavität verwendet werden.

Die Grenze zwischen niedrig Z-Material und hoch Z-Material liegt im Rahmen der vorliegenden Offenbarung vorzugsweise zwischen Ordnungszahl 70-50 und besonders bevorzugt bei 60. Auch wenn die beiden Bereiche für die Ordnungszahl 60 überlappen, ist das hoch Z-Material immer ein anderes als das niedrig Z-Material, um den unterschiedlichen Absorptionsanforderungen gerecht zu werden.

Beispiele für niedrig Z-Materialien sind Aluminium, Zinn, Antimon, Iod, Cäsium, Barium, Lanthan, Cer, Praseodym, Neodym. Eines oder mehrere dieser Elemente kann zusätzlich noch mit Elementen vermischt sein, die nicht aus dieser Gruppe sind, beispielsweise eignen sich Elemente der seltenen Erden mit Z=60 bis 70, vorzugsweise das Samarium, Gadolinium, Terbium, und/oder Erbium und/oder Ytterbium, um in eine solche Mischung mit eingesetzt zu werden.

Besonders bevorzugt als niedrig Z-Material ist auch die Verwendung von Chlor mit der Ordnungszahl Z=17. Chlor wird typischerweise in der Polymerchemie als Element mit hoher Ordnungszahl verwendet. Somit ist Chlor auch für die vorliegende Erfindung besonders bevorzugt.

Bei Materialien mit einer Ordnungszahl kleiner/gleich Chlor (Z=17), nimmt die Emission von Bremsstrahlung bei Verwendung als Betastrahlenschutzbrille zu. Somit kann der strahlenschutztechnische Effekt des niedrig Z-Materials zur Abschirmung von Betastrahlung in der Summe abnehmen. Aus diesem Grund werden vorzugsweise Materialien mit einer Ordnungszahl kleiner/gleich Chlor verwendet.

Als hoch Z-Materialien eignen sich beispielsweise Bismuth, Wolfram und Tantal. Auch Blei oder Bleiersatzmaterialien eignen sich als hoch Z-Material.

Die erste Materialschicht kann in dem Zwischenraum benachbart zur äußeren Seitenwand und die zweite Materialschicht in dem Zwischenraum benachbart zur inneren Seitenwand angeordnet sein.

Ein derartiger Schichtaufbau ist besonders bei der Verwendung der Brille als Betastrahlenschutzbrille vorteilhaft, wenn die erste Materialschicht aus einem niedrig Z-Material und die zweite Materialschicht aus einem hoch Z-Material besteht. Somit kann die außenliegende niedrig Z-Materialschicht auftreffende Betastrahlung absorbieren und die innenliegende hoch Z-Materialschicht hierdurch erzeugte sekundäre Bremsstrahlung absorbieren.

Der Begriff "Absorbieren" bedeutet nicht nur eine komplette Absorption der auftreffenden Strahlung, sondern auch eine teilweise Absorption der Strahlung, d.h. eine Schwächung der auftreffenden Strahlung.

Für die Verwendung als Betastrahlenschutzbrille und/oder als CT-Strahlenschutzbrille (Röntgenstrahlenschutzbrille) eignet sich beispielsweise eine Bleifolie (hoch Z-Material) mit einem vorbestimmten Bleigleichwert, insbesondere von 0,127 mm Pb, und einer Aluminiumfolie (niedrig Z-Material) mit 0,1 mm Dicke.

Die möglichen Abmessungen, wie Dicke der Schicht oder Schichten für die Strahlenschutzmaterialien werden entsprechend der Breite des Zwischenraums bestimmt. Bei einem Zwischenraum von 2 mm können beispielsweise Strahlenschutzmaterialien mit einer Dicke von etwa 1,6 mm (Einbautoleranzen abgezogen) eingefügt werden. Innerhalb dieser Dicke von 1,6 mm ist es möglich, z. B. zwei Strahlenschutzmaterialien, niedrig und hoch Z-Material, z.B. Folien, beliebig in der Dicke zu variieren. Vorzugsweise werden in diesem Beispiel Folien mit einer Dicke im Bereich von 0,05 mm und 1,55 mm verwendet, wobei die Summe der Dicken beider Folien vorzugsweise etwa 1,6 mm beträgt.

Wird der Zwischenraum im oben beschriebenen Gieß- oder Spritzgussverfahren befüllt, wird der Zwischenraum vorzugsweise vollständig gefüllt, wobei keine Toleranz für die Füllung notwendig ist.

Die Brille kann ferner ein für sichtbares Licht durchlässiges Element aufweisen, das vorzugsweise im Bereich des distalen Endes jeder Brillenfassung angeordnet ist.

Die innere Seitenwand kann dabei eine geringere Höhe in distaler Richtung aufweisen als die äußere Seitenwand, wobei das durchlässige Element im Wesentlichen bündig mit der äußeren Seitenwand abschließt und/oder am distalen Ende der inneren Seitenwand aufliegt.

Vorzugsweise ist das durchlässige Element mit den Seitenwänden chemisch dicht verbunden. Eine chemisch dichte Verbindung zwischen dem durchlässigen Element und den Seitenwänden kann durch Anlösen der Kunststoffe mit Lösungsmitteln an den jeweiligen Grenzflächen erreicht werden. Ein solches Verfahren ist auch als "Kaltverschweißen von Kunststoffen" bekannt. Dies hat den Vorteil, dass als Strahlenschutzmaterial Schwermetalle eingesetzt werden können.

Das lichtdurchlässige Element ermöglicht dem Patienten, während der Untersuchung oder Therapie durch die Strahlenschutzbrille hindurch zu sehen und Aktivitäten, wie z.B. Lesen, durchzuführen. Dies ist beispielsweise bei längeren Untersuchungen oder Therapien, wie z.B. der Betastrahlentherapie, besonders bevorzugt.

Das lichtdurchlässige Element kann ein Strahlenschutzmaterial, insbesondere niedrig Z-Materialien, insbesondere Plexiglas, oder mit hoch Z-Materialen beladene Kunststoffe, insbesondere Bismuth oder Bleiacryl, aufweisen.

Beispielsweise kann das lichtdurchlässige Element ein Kunststoffmaterial einer vorbestimmten Dicke, insbesondere 3 mm, aufweisen. Diese Dicke ist besonders vorteilhaft für einen Schutz vor Betastrahlern, da diese Dicke wirkungsvoll Betastrahlung bei einer geringen Produktion von Bremsstrahlung absorbiert.

Der Schutz vor Betastrahlung eines Kunststoffes ist im Wesentlichen durch die maximale Energie der Betastrahlung bestimmt. Wird Betastrahlung beispielsweise von Rhenium-188 emittiert (maximale Energie Betastrahlung = 2,1 MeV), ist die Dicke des Kunststoffes des lichtdurchlässigen Elements vorzugsweise 3 mm.

Im Allgemeinen schützt 1 mm Kunststoff vor ca. 1 MeV an kinetischer Energie der Betateilchen. So kann die Dicke des lichtdurchlässigen Materials anhand des spezifischen Betastrahlers angepasst werden. Der typische Energiebereich für Betastrahler liegt im Bereich von ca. 20 keV (³H) bis ca. 5,4 MeV (²⁰F). Die Dicke des lichtdurchlässigen Elements beträgt vorzugsweise zwischen etwa 20 µm und etwa 5,5 mm.

Alternativ kann das lichtdurchlässige Element, bei Verwendung in einer Schutzbrille für Röntgenstrahlung und/oder Gammastrahlung, auch Bleiacryl mit einer vorbestimmten Dicke, insbesondere 3 mm, bei einem vorbestimmten Bleigleichwert, insbesondere von 0,127 mm Pb, aufweisen. Diese Ausgestaltung ist besonders vorteilhaft zum Schutz vor primärer bzw. sekundärer Bremsstrahlung (auch Streustrahlung genannt) bis ca. 50 keV.

Dies ermöglicht einen effektiven Strahlenschutz von frontal auf die Augen eintreffender Strahlung. Somit kann ein effektiver Strahlenschutz aus allen Richtungen gewährleistet werden.

Die Brille kann ferner eine erste Öse aufweisen, die an dem der Nase zugewandten Teil der jeweiligen Seitenwandstruktur einer Brillenfassung vorgesehen ist, zur Befestigung des Verbindungselements, und/oder ferner eine zweite Öse aufweisen, die an dem dem der Nase zugewandten Teil gegenüberliegenden Teil der jeweiligen Seitenwandstruktur einer Brillenfassung vorgesehen ist, zur Befestigung des Halteelements.

Insbesondere ist das Halteelement zur Fixierung der Brille am Kopf des Patienten geeignet. Das Halteelement ist vorzugsweise aus einem elastischen Material, z.B. ein Elastomer, das von einer zweiten Öse der rechten Brillenfassung um den Kopf des Patienten durchgängig bis zu der zweiten Öse der linken Brillenfassung verläuft.

Alternativ kann das Halteelement ein im Wesentlichen steifes (starres) Material aufweisen, welches geeignet ist, an den Ohren des Patienten befestigt zu werden.

Das Halteelement kann auch ohne eine zweite Öse direkt an der Seitenwandstruktur befestigt sein.

Die Bereitstellung von ersten und zweiten Ösen ist bevorzugt, um das Verbindungselement bzw. das Halteelement leicht austauschen zu können.

Die Form und Größe des lichtdurchlässigen Elements ist vorzugsweise der Form und Größe des Innenumfangs der äußeren Seitenwand im Wesentlichen angepasst.

Die Seitenwandstruktur, insbesondere das proximale Ende der Seitenwandstruktur, kann eine ergonomisch an die Kopfform des Patienten im Bereich um die Augen anpasste Form und/oder Größe aufweisen.

Die vorliegende Offenbarung betrifft ferner die Verwendung der Brille wie vorstehend beschrieben als Strahlenschutzbrille für den Schutz von Augen eines Patienten vor augenschädlicher Strahlung, insbesondere β-Strahlung und/oder Röntgenstrahlung und/oder Gammastrahlung.

Die vorliegende Erfindung ist dabei besonders vorteilhaft für die Verwendung als Strahlenschutzmittel für Betaemitter, wie sie bei der Rhenium Tumortherapie verwendet werden. Bei der Rhenium Tumortherapie wird der Betastrahler offen auf die zu behandelnde Stelle, z.B. im Gesicht eines Patienten, gegeben, um den Tumor zu behandeln. Dabei wird vorzugsweise eine mit einem Betastrahler beladene Paste aufgetragen. Die von dem Betastrahler ausgesendete Strahlung sollte dabei nicht auf die Augen des Patienten treffen. Um die Augen des Patienten vor der emittierten Betastrahlung zu schützen, kann eine Strahlenschutzbrille wie vorstehend beschrieben verwendet werden.

Da diese Therapien typischerweise 30-60 min oder länger dauern können, ist ein möglichst festsitzender Sitz des Strahlenschutzmittels erforderlich. Ferner sollte dem Patienten die Möglichkeit geben werden, während der Behandlung zu lesen o.ä.

Radionuklide emittieren typischerweise nicht ausschließlich Betastrahlung, sondern zusätzlich auch Gammastrahlung. Dies erklärt sich damit, dass sich die Atome nach Aussenden der Betastrahlung noch in einem angeregten Zustand befinden und nach Emission der Gammastrahlung in den energetisch bevorzugten Grundzustand zurückfallen. Diese physikalische Eigenschaft wiederum hat grundlegende Konsequenzen auf die technische Umsetzung eines Strahlenschutzmittels für solche Betaemitter.

Typische Energien für die auftretenden Gammastrahlen sind höher als bei der diagnostischen Betastrahlung (>120 keV). Daher sollte eine Strahlenschutzbrille zur Verwendung als Betastrahlenschutzbrille sowohl vor Betastrahlung als auch vor niederenergetischer Brems- bzw. Gammastrahlung (<120 keV) schützen. Dies ist durch die Verwendung eines niedrig Z-Materials und eines hoch Z-Materials wie vorstehend beschrieben besonders vorteilhaft möglich.

Ferner kann eine so aufgebaute Brille, d.h. mit niedrig Z-Material und hoch Z-Material, auch für eine CT-Patientenschutzbrille verwendet werden, da das hoch Z-Material auch zum Schutz vor direkter Röntgenstrahlung geeignet ist.

Eine Kombination aus niedrig-Z-Material und hoch-Z-Material ist demzufolge aus mehreren Gründen für den Einsatz in der Rhenium Tumortherapie vorteilhaft. Kunststoff, respektive Aluminium (oder ein vergleichbares niedrig-Z-Material) schirmt wirkungsvoll vor Betastrahlung und minimiert dabei die Erzeugung von Bremsstrahlung. Gleichzeitig absorbiert die dem Auge näherliegende Schicht (hoch-Z-Material) effektiv die erzeugte Bremsstrahlung und die Gammastrahlungskomponente des Radioisotops.

Die gewählte technische Umsetzung der Strahlenschutzbrille ermöglicht es zusätzlich, abhängig von den in der Therapie eingesetzten Radioisotopen, die Schichtdicke für hoch-Z- und niedrig-Z-Material entsprechend der Energie der Strahlungsarten anzupassen.

Bei einer hochenergetischen Betakomponente kann es vorteilhaft sein, eine dickere Schicht niedrig-Z-Material zu verwenden. Während bei einer hochenergetischen Gammakomponente eine dickere Schicht hoch-Z-Material verwendet werden kann. Randbedingung für die Schichtdicken ist hierbei ausschließlich die zur Verfügung stehende Breite des Zwischenraums zwischen innerer und äußerer Seitenwand.

Die anhand des Betastrahlers beschriebenen Merkmale gelten analog auch für Röntgenstrahler.

Ferner ist die erfindungsgemäße Brille vorteilhaft, da die Brille durch ihre spezifische Ausgestaltung mit zwei Fassungen und einem dazwischen angebrachten Verbindungselement eine möglichst kleine Fläche einnimmt. Somit kann die Paste mit dem Betastrahler großflächig auf mit Tumor befallenen Hautpartien aufgetragen werden.

Nachfolgend wird die vorliegende Erfindung anhand von Ausführungsbeispielen und der Figuren näher erläutert.

### Kurze Figurenbeschreibung

Es zeigen:
Fig. 1a eine perspektivische Ansicht gemäß einer Ausführungsform einer Brillenfassung für eine erfindungsgemäße Brille,
Fig. 1b eine rückseitige Ansicht gemäß der Ausführungsform einer Brillenfassung für eine erfindungsgemäße Brille,
Fig. 1c eine erste Seitenansicht gemäß der Ausführungsform einer Brillenfassung für eine erfindungsgemäße Brille,
Fig. 1d eine zweite Seitenansicht gemäß der Ausführungsform einer Brillenfassung für eine erfindungsgemäße Brille,
Fig. 1e eine Vorderansicht gemäß der Ausführungsform einer Brillenfassung für eine erfindungsgemäße Brille, und
Fig. 2 eine schematische Zeichnung gemäß einer Ausführungsform einer erfindungsgemäßen Brille.

### Bevorzugte Ausführungsformen

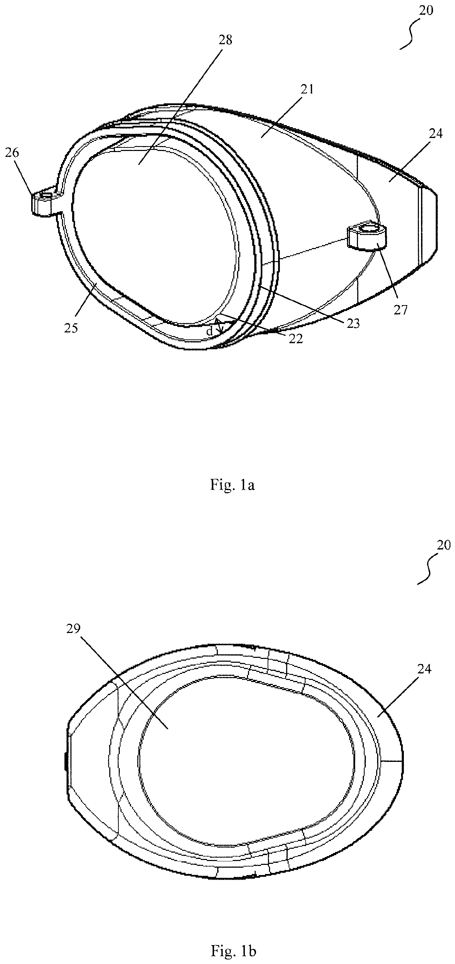

Mit Bezug auf die Figuren 1a-1e wird nachfolgend eine beispielhafte Ausführungsform einer Brillenfassung für eine Strahlenschutzbrille beschrieben.

Die in den Figuren 1a-1e gezeigte Brillenfassung entspricht einer Brillenfassung für das linke Auge eines Patienten. Die Beschreibung gilt aber entsprechend für eine Brillenfassung für das rechte Auge, wobei die Elemente dabei gegenüber der hier gezeigten Brillenfassung für das linke Auge gespiegelt sind, sodass die nachfolgend beschriebenen Verbindungselemente der Brillenfassungen für das rechte und das linke Auge sich gegenüberliegen, um mit einem Verbindungselement verbunden zu werden.

Figur 1a zeigt eine perspektivische Ansicht der Brillenfassung 20 gemäß einer beispielhaften Ausführungsform. Die Brillenfassung 20 weist eine Seitenwandstruktur 21 mit einer inneren Seitenwand 22 und einer äußeren Seitenwand 23 auf.

Die Seitenwandstruktur 21 bzw. die innere und die äußere Seitenwand 22, 23 weisen eine im Wesentlichen ovale Form auf, um einen Bereich um das Auge des Patienten seitlich möglichst vollständig abzudecken.

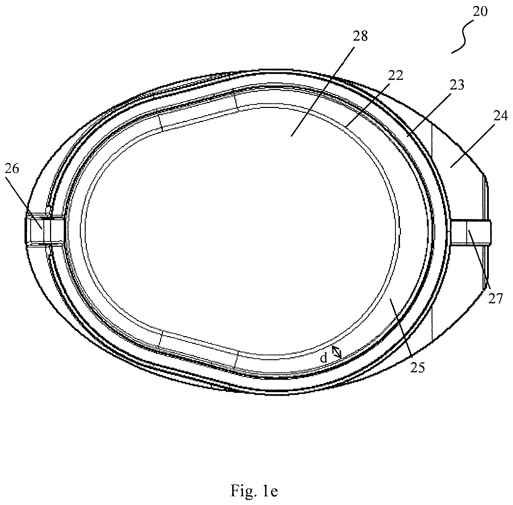

Die innere und die äußere Seitenwand bilden einen Zwischenraum 25 mit einem Abstand d. Der Abstand d des Zwischenraums kann beispielsweise in einem Bereich von 0,5 mm bis 2 mm liegen und vorzugsweise 1 mm betragen. Der Abstand variiert vorzugsweise über den Umfang der Brillenfassung 20. Vorzugsweise ist der Abstand wie nachstehend anhand von Fig. 1e beschrieben an der der Nase gegenüberliegenden Seite breiter als der Abstand an der der Nase zugewandten Seite.

Die Brillenfassung 20 weist ferner eine Basiswand 24 auf. Die Basiswand hat eine der um das Auge liegenden Fläche nachempfundene Form, um möglichst abschließend am Gesicht bzw. an der Haut des Patienten anzuliegen.

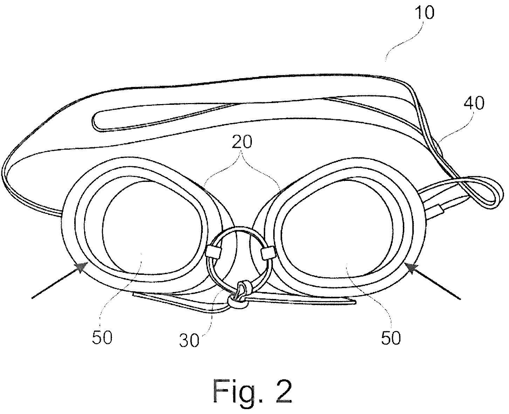

Ferner weist die Brillenfassung 20 eine erste Öse 26 auf, die an dem der Nase zugewandten Teil der Seitenwandstruktur 21 der Brillenfassung 20 vorgesehen ist. Die Öse 26 dient dabei der Befestigung eines Verbindungselements 30 (cf. Fig. 2).

Die Brillenfassung weist zusätzlich eine zweite Öse 27 auf, die an dem dem der Nase zugewandten Teil gegenüberliegenden Teil der Seitenwandstruktur 21 der Brillenfassung 20 vorgesehen ist. Die Öse 27 dient zur Befestigung eines Halteelements 40 (cf. Fig. 2).

Die Seitenwandstruktur 21 definiert eine erste Öffnung 28 am distalen Ende der Brillenfassung 20, durch die der Patient hindurchschauen kann. Die Öffnung 28 kann ein lichtdurchlässiges Element 50 (cf. Fig. 2) aufweisen.

Fig. 1b zeigt eine rückwärtige Ansicht der Brillenfassung 20 gemäß Fig. 1a. In Fig. 1b ist die Basiswand 24 besonders gut zu erkennen. Die Basiswand 24 befindet sich auf der Rückseite der Brillenfassung 20, d.h. der Seite, die am Gesicht des Patienten aufliegt, wenn dieser die Brille aufsetzt.

Die Basiswand 24 verbindet die innere und die äußere Seitenwand 22, 23 an deren proximalem Ende. Die Basiswand 24 definiert eine zweite Öffnung 29 am proximalen Ende der Brillenfassung 20, durch die der Patient hindurchschauen kann.

Die erste Öffnung 28 und die zweite Öffnung 29 sind in Richtung vom distalen zum proximalen Ende zueinander ausgerichtet, sodass es dem Patienten ermöglicht wird, durch beide Öffnungen 28, 29 hindurchzuschauen.

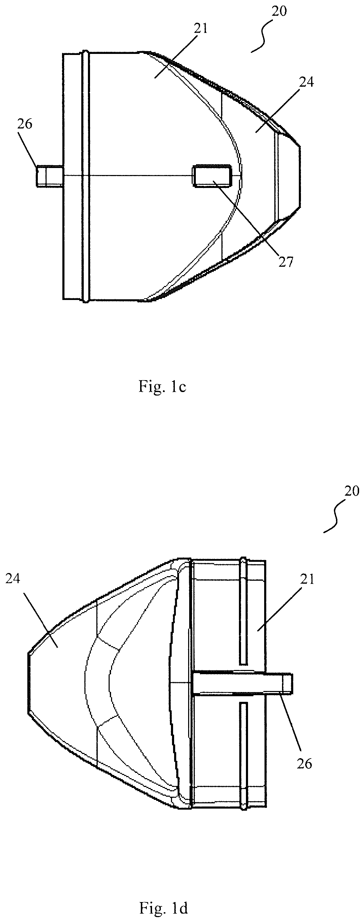

Fig. 1c zeigt eine Seitenansicht der beispielhaften Ausführungsform gemäß Fig. 1a. Fig. 1c zeigt die Seite der Brillenfassung 20, die der Nase eines Patienten abgewandt ist.

Insbesondere zeigt Fig. 1c die Seitenwandstruktur 21, die Basiswand 24, die erste und die zweite Öse 26, 27.

Die erste Öse 26 steht dabei nach vorne, d.h. am distalen Ende, der Seitenwandstruktur 21 über. Dies ermöglicht eine besonders vorteilhafte Anbringung eines Verbindungselements 30 (cf. Fig. 2) in einem gewissen Abstand zur Nase des Patienten.

Ferner ist die Anbringung der zweiten Öse 27 an der Seitenwandstruktur 21 in Fig. 1c gezeigt. Die zweite Öse 27 ist vorzugsweise im proximalen Bereich der Seitenwandstruktur 21 angeordnet, um eine vorteilhafte Fixierung der Brillenfassungen 20 am Kopf des Patienten zu ermöglichen.

Fig. 1d zeigt eine Seitenansicht der beispielhaften Ausführungsform gemäß Fig. 1a. Fig. 1d zeigt die Seite der Brillenfassung 20, die der Nase eines Patienten zugewandt ist.

Insbesondere zeigt Fig. 1d die Seitenwandstruktur 21, die Basiswand 24, und die erste Öse 26. Wie aus Fig. 1d ersichtlich, ist die Öse 26 an der Seitenwandstruktur 21 angebracht und steht am distalen Ende der Seitenwandstruktur 21 über die Seitenwandstruktur 21 nach vorne hinaus.

Fig. 1e zeigt eine Vorderansicht der beispielhaften Ausführungsform gemäß Fig. 1a. Insbesondere zeigt Fig. 1e die innere und die äußere Seitenwand 22, 23, die Basiswand 24, den Zwischenraum 25, und die erste und die zweite Öse 26, 27.

In Fig. 1e ist der Abstand d des Zwischenraums an der der Nase des Patienten abgewandten Seite der Brillenfassung 20 breiter als der Zwischenraum an der der Nase des Patienten zugewandten Seite der Brillenfassung 20. Der Zwischenraum wird dabei graduell zu der der Nase des Patienten abgewandten Seite der Brillenfassung 20 breiter.

Fig. 2 zeigt eine beispielhafte Ausführungsform der Brille, d.h. der Strahlenschutzbrille. Insbesondere zeigt Fig. 2 zwei Brillenfassungen 20, die wie vorstehend beispielhaft beschrieben ausgestaltet sind. Daher wird Bezug genommen auf die Beschreibung der Figuren 1a-1e.

Die Brille 10 gemäß Fig. 2 zeigt zusätzlich zu den Brillenfassungen 20 ein Verbindungselement 30, ein Halteelement 40 und ein lichtdurchlässiges Element 50.

Ferner ist durch die Pfeile das in den vorstehend beschriebenen Zwischenraum 25 (cf. Fig. 1a-1e) eingebrachte Strahlenschutzmaterial, vorzugsweise bestehend aus einem niedrig Z-Material und einem hoch Z-Material, identifiziert.

Das Verbindungselement 30 und das Halteelement 40 sind verstellbar, sodass die Brille 10 für verschiedene Patienten mit unterschiedlichen Kopfgrößen und Kopfformen, insbesondere unterschiedlichen Augenabständen, verwendet werden kann.

Das lichtdurchlässige Element 50 schließt die Öffnung 28 am distalen Ende der Seitenwandstruktur 21 ab und erlaubt dem Patienten während der Behandlung durch das lichtdurchlässige Element hindurchzuschauen.

Während die vorliegende Erfindung hier unter Bezug auf ihre bevorzugten Ausführungsformen beschrieben und dargestellt wurde, ist für Fachleute auf dem Gebiet offensichtlich, dass verschiedene Modifikationen und Änderungen daran vorgenommen werden können, ohne den Schutzbereich der Erfindung zu verlassen. Auf diese Weise ist beabsichtigt, dass die vorliegende Erfindung die Modifikationen und Änderungen dieser Erfindung abdeckt, sofern sie in den Schutzbereich der beigefügten Patentansprüche und ihrer Äquivalente fallen. Insbesondere ist dem Fachmann klar, dass die Merkmale, welche im Zusammenhang mit einer bestimmten bevorzugten Ausführungsform beschrieben wurden, auch mit Merkmalen anderer Ausführungsformen kombiniert werden können.

## Patentansprüche

1. Brille (10) für die Aufnahme von mindestens einem Strahlenschutzmaterial, das die Augen eines Patienten vor augenschädlicher Strahlung, insbesondere β-Strahlung und/oder Röntgenstrahlung und/oder Gammastrahlen, schützt, wobei die Brille (10) aufweist:
zwei Brillenfassungen (20), die jeweils eine durchgängig umlaufende Seitenwandstruktur (21) aufweisen, die einen Bereich um das jeweilige Auge des Patienten vollständig umschließen,
ein längenverstellbares Verbindungselement (30) zwischen den zwei Brillenfassungen (20) im Bereich der Nase des Patienten, um den Abstand der zwei Brillenfassungen (20) zueinander zu verändern, und
mindestens ein längenverstellbares Halteelement (40), um die zwei Brillenfassungen (20) am Kopf des Patienten zu fixieren, **dadurch gekennzeichnet, dass** die Seitenwandstrukturen (21) konfiguriert sind, mindestens ein Strahlenschutzmaterial im Wesentlichen vollumfänglich aufzunehmen, und
wobei die Seitenwandstruktur (21) jeweils eine innere und eine äußere Seitenwand (22, 23) aufweist, wobei die innere und die äußere Seitenwand (22, 23) einen vorbestimmten Abstand in Querrichtung zur Blickrichtung des Patienten aufweisen, um einen Zwischenraum (25) zu bilden, wobei der Zwischenraum (25) konfiguriert ist, das mindestens eine Strahlenschutzmaterial aufzunehmen.

2. Brille (10) nach Anspruch 1, wobei die innere und die äußere Seitenwand (22, 23) der Seitenwandstruktur (21) über eine Basiswand (24) an einem Ende der Seitenwandstruktur verbunden sind.

3. Brille (10) nach Anspruch 1 oder 2 mit mindestens einem Strahlenschutzmaterial, wobei das Strahlenschutzmaterial derart ausgebildet ist den Zwischenraum (25) in Längsrichtung vollständig auszufüllen.

4. Brille (10) nach einem der Ansprüche 1 bis 3 mit mindestens einem Strahlenschutzmaterial, wobei das mindestens eine Strahlenschutzmaterial eine erste Materialschicht aus einem Material mit niedriger Kernladungszahl Z, insbesondere Al, und/oder eine zweite Materialschicht aus einem Material mit hoher Kernladungszahl Z, insbesondere Blei oder Bleiersatzmaterialien, wie Bismuth, Wolfram, Tantal oder Verbindungen dieser Metalle, aufweist, wobei die erste Materialschicht und die zweite Materialschicht vorzugsweise in Form einer Verbundschicht, in Form einer dünnen Folie oder als Pulverform in einem Matrixmaterial dispergiert oder als Flüssigkeit zum Gießen oder Spritzen, vorgesehen sind.

5. Brille (10) nach Anspruch 4, wobei die erste Materialschicht in dem Zwischenraum (25) benachbart zur äußeren Seitenwand (23) und die zweite Materialschicht in dem Zwischenraum (25) benachbart zur inneren Seitenwand (22) angeordnet sind.

6. Brille (10) nach einem der Ansprüche 1 bis 5, mit einem für sichtbares Licht durchlässigen Element (50), das vorzugsweise im Bereich des distalen Endes jeder Brillenfassung (20) angeordnet ist, wobei vorzugsweise die innere Seitenwand (22) eine geringere Höhe in distaler Richtung aufweist als die äußere Seitenwand (23), wobei das durchlässige Element (50) im Wesentlichen bündig mit der äußeren Seitenwand (23) abschließt und/oder am distalen Ende der inneren Seitenwand (22) aufliegt.

7. Brille (10) nach Anspruch 6, wobei das durchlässige Element (50) ein Strahlenschutzmaterial, insbesondere niedrig Z-Materialien, insbesondere Plexiglas, oder mit hoch Z-Materialen beladene Kunststoffe, insbesondere Bismuth oder Bleiacryl, aufweist.

8. Brille (10) nach einem der Ansprüche 1 bis 7, ferner aufweisend eine erste Öse (26), die an dem der Nase zugewandten Teil der jeweiligen Seitenwandstruktur (21) einer Brillenfassung (20) vorgesehen ist, zur Befestigung des Verbindungselements (30) und/oder ferner aufweisend eine zweite Öse (27), die an dem dem der Nase zugewandten Teil gegenüberliegenden Teil der jeweiligen Seitenwandstruktur (21) einer Brillenfassung (20) vorgesehen ist, zur Befestigung des Halteelements (40).

9. Brille (10) nach einem der Ansprüche 6 bis 8, wobei die Form und Größe des durchlässigen Elements (50) der Form und Größe des Innenumfangs der äußeren Seitenwand (23) im Wesentlichen angepasst ist.

10. Brille (10) nach einem der Ansprüche 1 bis 9, wobei die Seitenwandstruktur (21), insbesondere das proximale Ende der Seitenwandstruktur (21), eine ergonomisch an die Kopfform des Patienten im Bereich um die Augen anpasste Form und/oder Größe aufweist.

## Claims

1. Goggles (10) for receiving at least one radiation protection material which protects the eyes of a patient from radiation being harmful to the eyes, in particular β-radiation and/or x-ray radiation and/or gamma radiation, wherein the goggles (10) comprise:
two goggle frames (20) each comprising a continuously circumferential side wall structure (21) completely enclosing an area around the respective eye of the patient,
a length-adjustable connection element (30) between the two goggle frames (20) in the area of the nose of the patient for changing the distance between the two goggle frames (20) relative to one another, and
at least one length-adjustable holding element (40) for fixing the two goggle frames (20) to the head of the patient,
**characterized in that**
the side wall structures (21) are configured for receiving at least one radiation protection material substantially completely around its circumference, and
wherein the side wall structure (21) comprises an inner and an outer side wall (22, 23),
wherein the inner and the outer side wall (22, 23) have a predetermined distance in the cross direction to the viewing direction of the patient in order to form a space (25), the space (25) being configured for receiving the at least one radiation protection material.

2. The goggles (10) according to claim 1, wherein the inner and the outer side wall (22, 23) of the side wall structure (21) are connected by means of a base wall (24) at an end of the side wall structure.

3. The goggles (10) according to claim 1 or 2 comprising at least one radiation protection material, wherein the radiation protection material configured so as to fill the space (25) completely in the longitudinal direction.

4. The goggles (10) according to any one of claims 1 to 3 comprising at least one radiation protection material, wherein the at least one radiation protection material comprises a first material layer formed of a material having a low atomic number Z, in particular Al, and/or a second material layer formed of a material having a high atomic number Z, in particular lead or lead replacement materials such as bismuth, tungsten, tantalum or compounds of these metals, wherein the first material layer and the second material layer are preferably provided as a compound layer, as a thin film or as a powder dispersed in a matrix material or as a liquid for casting or injection molding.

5. The goggles (10) according to claim 4, wherein the first material layer is arranged in the space (25) neighboring the outer side wall (23) and the second material layer is arranged in the space (25) neighboring the inner side wall (22).

6. The goggles (10) according to any one of claims 1 to 5, comprising a light transmissive element (50) which is preferably arranged in the area of the distal end of each goggle frame (20), wherein preferably the inner side wall (22) has a smaller height in the distal direction than the outer side wall (23), wherein the transmissive element (50) is substantially flush with the outer side wall (23) and/or rests against the distal end of the inner side wall (22).

7. The goggles (10) according to claim 6, wherein the light transmissive element (50) comprises a radiation protection material, in particular low-Z materials, in particular acrylic glass, or plastic materials loaded with high-Z materials, in particular bismuth or lead acrylic.

8. The goggles (10) according to any one of claims 1 to 7, further comprising a first eye (26) provided at the part of the respective side wall structure (21) of a goggle frame (20) facing the nose for attaching the connection element (30) and/or further comprising a second eye (27) provided at the part of the respective side wall structure (21) of a goggle frame (20) opposite the part facing the nose for attaching the holding element (40).

9. The goggles (10) according to any one of claims 6 to 8, wherein the shape and size of the light transmissive element (50) are preferably substantially adapted to the shape and size of the inner circumference of the outer side wall (23).

10. The goggles (10) according to any one of claims 1 to 9, wherein the side wall structure (21), in particular the proximal end of the side wall structure (21), has a shape and/or size being ergonomically adapted to the shape of the patient's head in the area around the eyes.

## Revendications

1. Lunettes (10) pour la réception d'au moins un matériau de protection contre les rayonnements qui protège les yeux d'un patient contre un rayonnement nocif pour les yeux, en particulier le rayonnement β et/ou le rayonnement X et/ou les rayons gamma, où les lunettes (10) présentent:
deux montures de lunettes (20), qui présentent chacune une structure de paroi latérale (21) continûment tournante, qui entourent complètement une zone autour de l'œil respectif du patient,
un élément de liaison réglable en longueur (30) entre les deux montures de lunettes (20) dans la zone du nez du patient, pour modifier la distance des deux montures de lunettes (20) l'une par rapport à l'autre, et
au moins un élément de maintien réglable en longueur (40) pour fixer les deux montures de lunettes (20) sur la tête du patient,
**caractérisées en ce que** les structures de paroi latérale (21) sont configurées pour recevoir au moins un matériau de protection contre les rayonnements sensiblement sur toute la périphérie, et
où la structure de paroi latérale (21) présente dans chaque cas une paroi latérale intérieure et une paroi latérale extérieure (22, 23), où la paroi latérale intérieure et la paroi latérale extérieure (22, 23) présentent une distance prédéterminée dans la direction transversale à la direction de vue du patient pour former un espace intermédiaire (25), où l'espace intermédiaire (25) est configuré pour recevoir le au moins un matériau de protection contre les rayonnements.

2. Lunettes (10) selon la revendication 1, où la paroi latérale intérieure et la paroi latérale extérieure (22, 23) de la structure de paroi latérale (21) sont reliées par une paroi de base (24) à une extrémité de la structure de paroi latérale.

3. Lunettes (10) selon la revendication 1 ou 2 avec au moins un matériau de protection contre les rayonnements, où le matériau de protection contre les rayonnements est conçu pour remplir complètement l'espace intermédiaire (25) dans la direction longitudinale.

4. Lunettes (10) selon l'une des revendications 1 à 3 avec au moins un matériau de protection contre les rayonnements, où le au moins un matériau de protection contre les rayonnements présente une première couche de matériau en un matériau à faible numéro atomique Z, en particulier Al, et/ou une seconde couche de matériau en un matériau à numéro atomique Z élevé, en particulier le plomb ou les matériaux de substitution du plomb tels que le bismuth, le tungstène, le tantale ou des composés de ces métaux, où la première couche de matériau et la seconde couche de matériau sont prévues de préférence sous forme d'une couche composite, sous forme d'un film mince ou sous forme de poudre dispersée dans un matériau de matrice ou sous forme de liquide à verser ou à pulvériser.

5. Lunettes (10) selon la revendication 4, où la première couche de matériau dans l'espace intermédiaire (25) est disposée adjacente à la paroi latérale extérieure (23) et la seconde couche de matériau dans l'espace intermédiaire (25) est disposée adjacente à la paroi latérale intérieure (22).

6. Lunettes (10) selon l'une des revendications 1 à 5, avec un élément perméable à la lumière visible (50), qui est de préférence disposé dans la zone de l'extrémité distale de chaque monture de lunettes (20), où de préférence la paroi latérale intérieure (22) présente une hauteur inférieure dans la direction distale à celle de la paroi latérale extérieure (23), où l'élément perméable (50) se termine sensiblement au ras de la paroi latérale extérieure (23) et/ou repose sur l'extrémité distale de la paroi latérale intérieure (22).

7. Lunettes (10) selon la revendication 6, où l'élément perméable (50) présente un matériau de protection contre les rayonnements, en particulier des matériaux à faible Z, en particulier du Plexiglas, ou des matières synthétiques chargées de matériaux à Z élevé, en particulier du bismuth ou de l'acrylique au plomb.

8. Lunettes (10) selon l'une des revendications 1 à 7, présentant en outre un premier œillet (26), qui est prévu sur la partie de la structure de paroi latérale respective (21) d'une monture de lunettes (20) tournée vers le nez, pour la fixation de l'élément de liaison (30) et/ou présentant en outre un second œillet (27) qui est prévu sur la partie de la structure de paroi latérale respective (21) d'une monture de lunettes (20) opposée à la partie tournée vers le nez, pour la fixation de l'élément de maintien (40).

9. Lunettes (10) selon l'une des revendications 6 à 8, où la forme et la taille de l'élément perméable (50) sont sensiblement adaptées à la forme et à la taille de la périphérie intérieure de la paroi latérale extérieure (23).

10. Lunettes (10) selon l'une des revendications 1 à 9, où la structure de paroi latérale (21), en particulier l'extrémité proximale de la structure de paroi latérale (21), présente une forme et/ou une taille ergonomiquement adaptée à la forme de la tête du patient dans la zone autour des yeux.
